(19) Europäisches Patentamt
European Patent Office
Office européen des brevets

(11) EP 2 074 997 A1

(12) **EUROPEAN PATENT APPLICATION**

(43) Date of publication:
**01.07.2009 Bulletin 2009/27**

(21) Application number: **09158047.2**

(22) Date of filing: **28.04.2003**

(51) Int Cl.:
*A61K 31/445* (2006.01) *A61K 31/40* (2006.01)
*A61K 31/485* (2006.01) *A61K 31/135* (2006.01)
*A61P 3/04* (2006.01)

(84) Designated Contracting States:
**AT BE BG CH CY CZ DE DK EE ES FI FR GB GR HU IE IT LI LU MC NL PT RO SE SI SK TR**

(30) Priority: **17.05.2002 EP 02011047**

(62) Document number(s) of the earlier application(s) in accordance with Art. 76 EPC:
**03752716.5 / 1 505 974**

(71) Applicant: **Tioga Pharmaceuticals, Inc.**
**San Diego, CA 92121 (US)**

(72) Inventors:
- **Weber, Frank**
**63128, Dietzenbach/Hexenberg (DE)**
- **Jacob, Jutta**
**56323, Waldesch (DE)**
- **Barber, Andrew**
**64331, Weiterstadt (DE)**
- **Gottschlich, Rudolf**
**64354, Reinheim (DE)**

(74) Representative: **Viering, Jentschura & Partner**
**Grillparzerstrasse 14**
**81675 München (DE)**

Remarks:
This application was filed on 16-04-2009 as a divisional application to the application mentioned under INID code 62.

# (54) Use of Asimadoline for the treatment of digestive disorders

(57) The instant invention relates to the use of compounds that are effective as selective opiate receptor modulators for the manufacture of pharmaceuticals for the diagnosis and/or the treatment of disorders, said disorders being selected from eating disorders and digestive disorders, especially psychogenic eating disorders, for the manufacture of a pharmaceutical effective for modulating the gastrointestinal tonus, and to pharmaceutical composition, comprising one or more of said modulator compounds and one or more compounds that are effective as appetite depressant.

Figure 1

Maximum volume ingested / ml (higher is better)

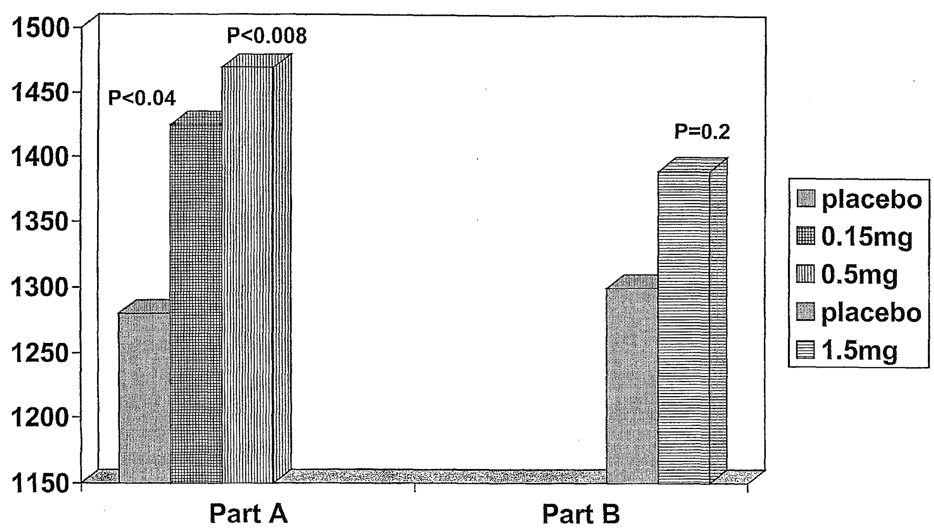


EP 2 074 997 A1

## Description

The instant invention relates to the use of compounds that are effective as selective opiate receptor modulators for the manufacture of pharmaceuticals for the diagnosis, prophylaxis and/or the treatment of disorders, said disorders being selected from eating disorders and digestive disorders, especially for psychogenic eating disorders, for the manufacture of a pharmaceutical effective for modulating the gastrointestinal tonus, and to pharmaceutical compositions, comprising one or more of said modulator compounds and one or more compounds that are effective as appetite depressant.

In civilized societies, the working and living conditions are more and more associated with all kinds of stress that lead in many cases to stress-related disorders. One major group of disorders that is believed to be at least partly induced or influenced by the modern living conditions and the stress associated there with is the group consisting of eating disorders and digestive disorders, especially psychogenic eating- and digestive disorders. Usually, this disorders are treated with psychotherapy and/or pharmaceutical preparations, that interact with the central nervous system. The treatment with such pharmaceutical preparations can lead to serious adverse effects such as habituation and addiction.

It was therefore object of the instant invention to make available pharmaceutically active compounds which can be used for the successful treatment of eating disorders and digestive disorders, especially psychogenic eating disorders and psychogenic digestive disorders. These pharmaceutically active compounds should be advantageous over prior art and, in particular, show little or no negative interaction with the central nervous system of the patient treated therewith.

Surprisingly, it was found that compounds that are effective as selective opiate receptor modulators and especially compounds that are effective as peripherally selective opiate receptor modulators can be successfully used in the treatment of eating disorders and digestive disorders. More surprisingly it was found that these compounds are capable of modulating the tonus of the gastrointestinal (GI) tract of the patient treated therewith highly effective, especially after GI-surgery. Even more surprisingly, it was found that the modulation the tonus of the GI tract of the patient can be advantageously controlled dosis dependent, i. e. the desired relaxation or activation of the GI tract, respectively, can be achieved depending on the dosis of the respective compound administered to the patient.

Accordingly, subject of the present invention is the use of a compound that is effective as selective opiate receptor modulator, preferably as selective opiate receptor agonist, for the manufacture of a pharmaceutical for diagnosis and/or the treatment of disorders, said disorders being selected from eating disorders and digestive disorders. Preferably, said receptor modulator is peripherally selective to the receptor. Especially preferred, said opiate receptor is a kappa-opiate receptor.

A preferred aspect of the instant invention therefore relates to the use of a compound that is effective as peripherally selective opiate receptor modulator for the manufacture of a pharmaceutical for diagnosis and/or the treatment of disorders, said disorders being selected from eating disorders and digestive disorders. A more preferred aspect of the instant invention relates to the use as described above, further **characterized in that** the compound is effective as peripherally selective opiate receptor agonist. An even more preferred aspect of the instant invention relates to the use as described above, further **characterized in that** the compound is effective as peripherally selective kappa-opiate agonist.

The compounds for use according to the invention preferably show one or more of the following advantageous properties:

- the compounds for use according to the invention are effective to modulate the tonus of the GI tract; especially they can be used to induce a relaxation or activation of the GI tonus; in general the modulation of the tonus of the GI tract is dosis dependent;
- the compounds for use according to the invention are effective to modulate satiety and/or postprandial symptoms, i. e , for example the amount of bloating, and the sensation of fullness, nausea and/or pain after ingestion of food;
- the effect on satiety and/or postprandial symptoms is preferably dosis dependent; in general lower doses lead to a decrease of symptoms, whereas higher doses can increase the symptoms;
- the compounds for use according to the invention are effective to modulate the fasting volume and/or the compliance of the GI tract and especially of the colon; for example, the fasting volume can be significantly increased by administration of lower to medium doses, compared to no administration;
- in general, no relevant effects on functional perimeters of the GI tract, such as the GI transit time, gastric emptying, intestinal and colonic emptying, can be observed; this effect is preferably not or little doses dependent; thus administration of a modulating compound does not affect the natural function of the GI tract and therefore shows only little tendency to induce unwanted adverse effects
- preferably, at higher doses the compounds according to the present invention increase symptom severity of gastric fullness and may therefore correct a missing signal in obese patients, i.e. the patients receive a signal of having a full stomach with lower volume ingested and thus eat less.

The dosis dependency of the effects on the GI tract on administration of compounds for use according to the invention can readily be determined according or analogously to methods known in the art, for example according to the method described herein. According to the invention, lower doses are in many cases in the range of about 0.001 to about 0.5 mg/kg daily, preferably about 0.01 to about 1.0 mg/kg daily and especially about 0.1 to about 2.0 mg/kg daily, for example at about 0.3 mg/kg daily, about 0.75 mg/kg daily or about 1.0 mg/kg daily, whereas higher doses lie usually above about 2.0 mg/kg daily preferably in the range of about 2.25 to about 5 mg/kg and especially in the range of about 2.5 mg/kg to about 10 mg/kg daily, for example at about 3 mg/kg daily, about 5 mg/kg daily or about 8 mg/kg daily.

The invention thus relates to the use of a compound that is effective as selective opiate receptor modulator, especially as peripherally selective opiate receptor modulator, for the manufacture of a pharmaceutical effective for modulating the gastrointestinal tonus.

The invention further relates to the use of a compound that is effective as selective opiate receptor modulator, especially as peripherally selective opiate receptor modulator, for the manufacture of a pharmaceutical for diagnosis and/or the treatment of disorders, said disorders being selected from eating disorders and digestive disorders, especially psychogenic eating disorders and digestive disorders.

Eating disorders and digestive disorders according to the invention comprise, but are not limited to, the regulation of pathological imbalanced appetite, loss of appetite or diminished appetite, induced for example by pregnancy, cancer, infection diseases like influenza or HIV, as a postoperatively adverse effect, as a result of catabolism, cachexy, anorexia, especially anorexia nervosa, dysorexia, dysponderosis, adiposity, bulimia, obesity, gastroparesis, especially neurogenic gastroparesis, diabetic gastroparesis, myogenic gastroparesis or gastroparesis induced by drugs, gastroatonia, gastroparalysis or enteroparesis, especially after GI-surgery, and stenosis of the gastrointestinal tract, especially stenosis of the pylorus.

A preferred embodiment of the instant invention therefore relates to the use of a compound that is effective as selective opiate receptor modulator, especially as peripherally selective opiate receptor modulator, for the manufacture of a pharmaceutical for the treatment of disorders selected from group consisting of regulation of pathological imbalanced appetite, anorexia, adiposity, bulimia, obesity, gastroparesis and stenosis of the gastrointestinal tract and especially anorexia nervosa, bulimia, obesity, diabetic gastroparesis and stenosis of the pylorus.

Compounds that are effective as selective opiate receptor modulators, especially as peripherally selective opiate receptor modulators, or more precisely, compounds that show selective activity against opiate receptors especially peripheral opiate receptors, are known to the skilled artisan and have been extensively described in the literature. These modulators are commonly divided into opiate receptor agonists and opiate receptor antagonists. Over the years, different subtypes of opiate receptors have been found and studied in detail, the kappa-opiate receptor (or $\kappa$-opiate receptor) and the mu-opiate receptor (or $\mu$-opiate receptor) belonging to the most prominent.

Suitable for use according to the invention are compounds that are effective as selective opiate modulators, preferably as peripherally selective opiate modulators, more preferably peripherally selective opiate agonists, even more preferred peripherally selective kappa- or mu-opiate agonists and especially preferred peripherally selective kappa-opiate agonists. These compounds are referred to hereinafter as "compounds for use according to the invention" or as "modulating compounds".

Various such modulating compounds are known in the art, for example from the subsequent cited literature:

DE-A1-3935371; DE 40 34 785, DE-A-4215231; EP-A-0569 802; EP 0 752 246; J. N. Sengupta et al., Pain 79 (1990) 175-185; Laurent Diop et al., European Journal of Pharmacology, 271 (1994) 65-71; Gottschlich et al., Chirality 6: 685-689 (1994); Gottschlich et al., Drugs Exptl. Clin. Res. XXI (5), 171-174 (1995); A. Barber et al., Br. J. Pharmacol. (1994), 113, 1317-1327; and J. N. Junien, P. Riviere, Aliment. Pharmacol. Ther 1995,: 9: 117-126; and the literature cited in the above referenced publications, which are both incorporated into the disclosure of the instant invention by reference.

The modulating compounds disclosed in the above cited references are included into this application by reference. Accordingly, the use of these modulating compounds for the manufacture of a pharmaceutical according to the invention is claimed subject matter of the present invention.

Further compounds for use according to the invention can be readily determined by the skilled artisan, for example by methods known and established in the art or analogously to these established methods, for example by receptor-binding assays, high throughput screening, *in vitro* testing-systems, *in vivo*-testing systems, animal models and the like. Examples for methods that can be used to identify compounds for use according to the invention are cited hereinafter:

Krimmer, E. C. et al., Fed. Proc. 1982 (5), 41(7): 2319-22; Spetea et al., Life Sciences 69 (2001), 1775-1782 and Lathi et al., European Journal Pharmacology 1985, 109: 281-284; and the literature cited in the above referenced publications, which are both incorporated into the disclosure of the instant invention by reference.

In general, compounds are to be regarded suitable as selective opiate receptor modulators for use according to the invention, i. e. modulating compounds, if they show an affinity to one or more opiate receptor, preferably to the mu- and kappa-opiate receptor, more preferably to the mu- or the kappa-opiate receptor and especially to the kappa-opiate receptor that lies, determined as $IC_{50}$-value, in the range of 100 μmol or below, preferably 10 μmol or below, more preferably in the range of 3 μmol or below, even more preferably in the range of 1 μmol or below and most preferably in the nanomolar range. Especially preferred for use according to the invention are opiate receptor modulators as defined above/below, that are peripherally selective acting opiate receptor modulators. In many cases an $IC_{50}$-value at the lower end of the given ranges is advantageous and in some cases its highly desirable that the $IC_{50}$-value is as small as possible, but in general $IC_{50}$-values that lie between the above given upper limits and a lower limit in the region of 0.0001 μmol 0.001 μmol, 0.01 μmol or even above 0.1 μmol are sufficient to indicate the desired pharmaceutical activity.

The meaning of peripherally selective activity of a compound, preferably of a pharmaceutically active compound or of a pharmaceutical containing such a compound, is known in the art and can be readily determined according to known procedures.

A peripherally selective compound according to the invention preferably means a compound that shows a selectivity for the peripheral nervous system when interacting with the body and preferably with the nervous system of the patient when administered to said patient. Peripherally selective compounds preferably thus show little or even more preferably no detectable impact on the central nervous system of the patient upon administration to said patient.

Preferred compounds for use according to the invention are compounds of formula I

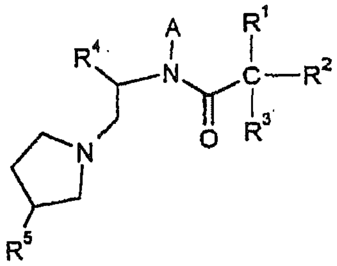

, (I)

in which

| | |
|---|---|
| $R^1$ | is Ar, cycloalkyl having 3-7 C atoms or cycloalkylalkyl having 4-8 C atoms, |
| $R^2$ | is Ar, |
| $R^1$ and $R^2$ | together are also |

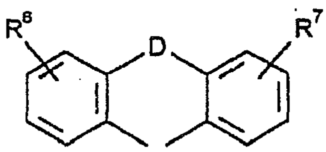

,

| | |
|---|---|
| $R^3$ | is H, OH, OA or A, |
| $R^4$ | is A or phenyl which can optionally be mono- or disubstituted by Hal, OH, OA, $CF_3$, $NO_2$, $NH_2$, NHA, NHCOA, $NHSO_2A$ or $NA_2$, |
| $R^5$ | is OH, $CH_2OH$, |
| $R^6$ and $R^7$ | in each case independently of one another are H, Hal, OH, OA, $CF_3$, $NH_2$, NHA, $NA_2$, NHCOA, $NHCONH_2$, $NO_2$ or methylenedioxy, |
| A | is alkyl having 1-7 C atoms, |
| Ar | is a mono- or bicyclic aromatic radical which can optionally contain an N, O or S atom and can be mono-, di- or trisubstituted by A, Hal, OH, OA, $CF_3$, $NH_2$, NHA, $NA_2$, NHCOA and/or $NHCONH_2$, |
| D | is $CH_2$, O, S, NH, NA, $-CH_2-CH_2-$, -CH=CH-, $-CH_2NH-$, $-CH_2-NA-$ or a bond |

and

Hal is F, Cl, Br or I,

and/or the salts and/or pharmaceutical acceptable derivatives thereof, and especially compounds of the formula I in which

Ar is phenyl,
$R^3$ is H,

and

A is methyl,

and/or the salts and/or pharmaceutical derivatives thereof, are pharmaceutically active compounds which are very particularly suitable as peripherally selective opiate receptor modulators for use according to the invention. Especially preferred as compound of the formula I is N-methyl-N-[(1S)-1-phenyl-2-((3S)-3-hydroxypyrrolidin-1-yl)ethyl]-2,2-diphenylacetamide (EMD 61753) and/or a salt and/or a pharmaceutical derivative thereof, preferably a pharmaceutical acceptable salt and especially the hydrochloride salt. This compound is known as Asimadoline.

Other preferred modulating compounds for use according to the invention are selected from a group consisting of of Alvimopan (see for example Am. J. Surg. 2001 Nov;182(5ASuppl):27S-38S), Loperamide (see for example J Pharmacol Exp Ther 1999 Apr;289(1):494-502), Spiradoline (see for example Pol. J. Pharmacol. 1994 Jan-Apr;46(1-2): 37-41), Fedotozine (see for example Expert Opin Investig Drugs. 2001 Jan;10(1):97-110), Pentazocine (see for example Biol Pharm Bull. 1997 Nov;20(11):1193-8), ICI204448 (see for example Br J Pharmacol. 1992 Aug;106(4):783-9), U-50488H (see for example Life Sci. 2002 Mar 1;70(15):1727-40), ADL 10-0101 (see for example Pain 2002 Mar;96(1-2): 13-22), ADL 10-0116 (see for example Pain 2002 Mar;96(1-2):13-22) and ADL 1-0398 (from Adolor Corp., USA)

In one preferred embodiment of the invention the modulating compounds are selected from a group consisting of Alvimopan, Loperamide, Fedotazine and Asimadoline.

In another preferred embodiment of the invention the modulating compounds are selected from a group consisting ICI204448, U-50488H, ADL 10-0101, ADL 10-0116 and ADL 1-0398.

In a more preferred embodiment of the invention, the modulating compounds are selected from a group consisting of Alvimopan, Loperamide, Asimadoline, ADL 10-0116 and ADL 1-0398.

Especially preferred for use according to the invention is Asimadoline or a salt or solvate thereof.

According to the invention, the term "pharmaceutical for the diagnosis of disorders" comprises pharmaceuticals that are used directly for diagnostic purposes as well as pharmaceuticals that enable or facilitate the application of diagnostic methods, for example by influencing the sensitivity, especially the sensitivity to pressure and pain, and/or the tonus of the gastrointestinal tract. In many cases, influencing or modulating of the tonus of the gastrointestinal tract leads to relaxation or activation of the gastrointestinal tonus and preferably to temporarily relaxation or activation of the gastrointestinal tonus. A modulation of the gastrointestinal tonus is advantageous for the application of most common diagnostic methods for the GI tract, such as endoscopic diagnostic methods and especially rectoscopy, endoscopic biopsy, endosonography and endoscopic x-ray methods. In many cases, influencing the gastrointestinal tonus is also advantageous to carry out surgery to the GI tract, especially if endoscopicical methods are used.

Thus, the use of compounds that are selective opiate receptor modulators (as described above) for the manufacture of a pharmaceutical for the supportive therapy of injuries, wounds or surgical lesions of the GI tract, for example from anal fissures, post recto-anal surgery and especially haemorrhoidectomy, is subject matter of the instant invention.

The compounds for use according to the invention are additionally advantageous as they preferably do not pass the blood-brain barrier or only to a minor, not relevant extent. This minimizes the risks of unwanted adverse effects.

Furthermore the compounds for use according to invention do not, or only to a minor, not relevant extent, interact with the Central nervous system of the patient they are administered to.

Since the compounds for use according to the invention are effective to increase postprandial symptoms, especially when administered at higher doses, they can be used as appetite depressant.

As the compounds for use according to the invention preferably do not interact with the central nervous system, it can be highly advantageous to combine them with conventional appetite depressants, preferably with appetite depressants that are effective by affecting the central nervous system, and especially with sympathomimetica, in the treatment of disorders that relate to excessive intake or ingestion of food and especially in the treatment of obesity or adipositas. A combination therapy comprising administering compounds for use according to the invention and conventional appetite depressants can be realized by administering two or more separate pharmaceutical preparations, each containing only one class of active ingredients, either a modulating compound affecting the peripheral nervous system or a conventional appetite depressant, affecting the central nervous system. On the other hand, a combination therapy can be realized by administering one pharmaceutical composition that contains both classes of active ingredients, one or more modulating compounds affecting the peripheral nervous system and one or more conventional appetite depressants and, if desired, one or more further ingredients, selected from the group consisting of additional active ingredients, excipients and

auxiliaries.

Thus, another aspect of the instant invention relates to the use of a compound that is effective as a selective opiate receptor modulator, especially effective as peripherally selective opiate receptor modulator, for the manufacture of a pharmaceutical to be administered in combination with one or more pharmaceuticals that are effective as an appetite depressant, preferably appetite depressants affecting the central nervous system.

Preferred conventional appetite depressants are selected from a group consisting of Phenylpropanolamin, Cathin, Sibutramin, Amfepramon, Ephedrin and Norpseudoephedrin of the salts thereof and especially Phenylpropanolamin hydrochloride, Cathin hydrochloride, Sibutramin hydrochloride, Amfepramon hydrochloride, Ephedrin hydrochloride and Norpseudoephedrin hydrochloride. The conventional appetite depressants listed above are usually referred to as sympathomimetica.

A preferred embodiment of this aspect of the instant invention relates to the use of one or more compounds selected from the group consisting of Alvimopan, Loperamide, Asimadoline, Fedotozine, Pentazocine, ICI204448, U-50488H, ADL 10-0101, ADL 10-0116 and ADL 1-0398 and especially the use of Asimadoline for the manufacture of the medicament to be used in combination with an appetite depressant, preferably selected from a group consisting of Phenylpropanolamin, Cathin, Sibutramin, Amfepramon, Ephedrin and Norpseudoephedrin.

Another aspect of the invention relates to pharmaceutical composition, comprising one or more compounds effective as a selective opiate receptor modulator and especially effective as a peripherally selective opiate receptor modulator, and one or more compounds that are effective as an appetite depressant, preferably one or more conventional appetite depressant and especially one or more sympathomimetica. Preferred are pharmaceutical compositions as described above wherein the selective opiate receptor modulator is selected from a group consisting of Alvimopan, Loperamide, Asimadoline, Fedotazine, Pentazocine, ICI204448, U-50488H, ADL 10-0101, ADL 10-0116 and ADL 1-0398 and/or the conventional appetite depressant is selected from group consisting of Phenylpropanolamin, Cathin, Sibutramin, Amfepramon, Ephedrin and Norpseudoephedrin or a salt thereof. Especially preferred are pharmaceutical compositions as described above wherein the selective opiate receptor modulator is selected from a group consisting of Alvimopan, Loperamide, Asimadoline, Fedotazine, ADL 10-0116 and ADL 1-0398 and especially is Asimadoline and/or or the conventional appetite depressant is selected from group consisting of Phenylpropanolamin, Cathin, Sibutramin, Amfepramon, Ephedrin and Norpseudoephedrin, or a salt thereof.

One special and preferred aspect of the invention relates to a pharmaceutical composition comprising Asimadoline and at least one appetite depressant, preferably a conventional appetite depressant and especially preferred at least one sympathomimeticum.

Another aspect of the invention relates to the use of a pharmaceutical composition as described above for the treatment of diseases, said diseases being selected from the group consisting of regulation of pathological imbalanced appetite, cachexy, anorexia, dysorexia, dysponderosis, adiposity, bulimia, obesity, gastroparesis, gastroatonia, gastroparalysis and stenosis of the gastrointestinal tract. In this aspect, the diseases are preferably selected from group consisting of regulation of pathological imbalanced appetite, adiposity or obesity.

Thus, the invention also relates to the use of a compound as defined in one of the claims 1 to 5, for the manufacture of a pharmaceutical to be used (to be administered) in combination with one or more pharmaceuticals that are effective as an appetite depressant.

In all indication areas described here, in particular the use of N-methyl-N-[(1S)-1-phenyl-2-((3S)-3-hydroxypyrrolidin-1-yl)ethyl]-2,2-diphenylacetamide hydrochloride (Asimadoline) as modulating compound and thus as a pharmaceutical or as active ingredient in a pharmaceutical has emerged as particularly effective. This particular high efficiency of asimadoline in all indications described herein is preferably maintained in all sorts of preparation forms.

Compounds for use according to the present invention are preferably selected from compounds which cannot pass through the blood-brain barrier on account of their structure and therefore do not exhibit a dependence potential. Also, until now no actions have been found which would restrict the use of the advantageous actions for the claimed indications in any way.

The compounds for use according to the present invention and/or their physiologically acceptable salts and/or their physiologically acceptable derivatives can therefore be used for the production of pharmaceutical compositions or preparations by bringing them into the suitable dose form together with at least one excipient or auxiliary and, if desired, with one or more further active compounds. The compositions or preparations thus obtained can be employed as medicaments in human or veterinary medicine. Suitable excipients are organic or inorganic substances which are suitable for enteral (e.g. oral or rectal) or parenteral administration and do not react with the compounds for use according to the present invention, for example water, vegetable oils, benzyl alcohols, polyethylene glycols, glycerol triacetate and other fatty acid glycerides, gelatin, soya lecithin, carbohydrates such as lactose or starch, magnesium stearate, talc or cellulose.

For oral administration, in particular tablets, coated tablets, capsules, syrups, juices or drops are used. Of interest are especially coated tablets and capsules having enteric coatings or capsule shells. For rectal administration, suppositories are used, and for parenteral administration, solutions, preferably oily or aqueous solutions, and also suspensions, emulsions or implants are used.

The compounds for use according to the invention can also be lyophilized and the lyophilisates obtained used, for example, for the production of injection preparations.

The compositions or preparations indicated can be sterilized and/or contain auxiliaries such as preservatives, stabilizers and/or wetting agents, emulsifiers, salts for affecting the osmotic pressure, buffer substances, colourants and/or flavourings. If desired, they can also contain one or more further active compounds, e.g. one or more vitamins, diuretics, anti-inflammatory or other compounds that can modulate the tonus of the Gi tract that are not selective opiate receptor modulators.

If the compound for use according to the invention is a compound with basic properties, it is usually called a base or free base of the compound. It can be advantageous to convert the free base into the associated acid-addition salt using an acid, for example by reaction of equivalent amounts of the base and the acid in an inert solvent, such as ethanol, followed by evaporation. The Suitable acids for this reaction are, in particular, those which give physiologically acceptable salts. Thus, it is possible to use inorganic acids, for example sulfuric acid, sulfurous acid, dithionic acid, nitric acid, hydrohalic acids, such as hydrochloric acid or hydrobromic acid, phosphoric acids, such as, for example, orthophosphoric acid, sulfamic acid, furthermore organic acids, in particular aliphatic, alicyclic, araliphatic, aromatic or heterocyclic monobasic or polybasic carboxylic, sulfonic or sulfuric acids, for example formic acid, acetic acid, propionic acid, hexanoic acid, octanoic acid, decanoic acid, hexadecanoic acid, octadecanoic acid, pivalic acid, diethylacetic acid, malonic acid, succinic acid, pimelic acid, fumaric acid, maleic acid, lactic acid, tartaric acid, malic acid, citric acid, gluconic acid, ascorbic acid, nicotinic acid, isonicotinic acid, methane- or ethanesulfonic acid, benzenesulfonic acid, trimethoxybenzoic acid, adamantanecarboxylic acid, p-toluenesulfonic acid, glycolic acid, embonic acid, chlorophenoxyacetic acid, aspartic acid, glutamic acid, proline, glyoxylic acid, palmitic acid, parachlorophenoxyisobutyric acid, cyclohexanecarboxylic acid, glucose 1-phosphate, naphthalenemono- and-disulfonic acids or laurylsulfuric acid. Salts with physiologically unacceptable acids, for example picrates, can be used to isolate and/or purify the compounds of the formula I. On the other hand, compounds of the formula I can be converted into the corresponding metal salts, in particular alkali metal salts or alkaline earth metal salts, or into the corresponding ammonium salts, using bases (for example sodium hydroxide, potassium hydroxide, sodium carbonate or potassium carbonate). Suitable salts are furthermore substituted ammonium salts, for example the dimethyl-, diethyl- and diisopropylammonium salts, monoethanol-, diethanol- and diisopropanolammonium salts, cyclohexyl- and dicyclohexylammonium salts, dibenzylethylenediammonium salts, furthermore, for example, salts with arginine or lysine.

Alternatively, compounds for use according to the invention with acidic properties can be converted into the associated base-addition salt using a base, for example by reaction of equivalent amounts of the acidic compound and the base in an inert solvent, such as ethanol, followed by evaporation. Examples for suitable bases are physiologically acceptable amines, hydroxides or carbonates, such as ethanol amine, sodium hydroxide, potassium hydroxide, sodium carbonate and potassium carbonate - oder Kaliumhydroxid oder -carbonat), that transfer the compounds for use according to the invention into the respective ammonium salts or metal salts.

On the other hand, if desired, the free bases of the formula I or the formula II can be liberated from their salts using bases (for example sodium hydroxide, potassium hydroxide, sodium carbonate or potassium carbonate).

Pharmaceutically acceptable derivatives of compounds for use according comprise prodrugs, metabolites and the like. Examples for such prodrugs and/or metabolites comprise compounds for use according to the invention that are modified with groups that are readily degraded/removed, such as alkyl groups, acyl groups and/or biodegradable polymers, and therefore liberate the compound for use according to the invention from the respective derivative. Examples for suitable biopolymers are described in the literature, for example Int. J. Pharm. 115, 61-67 (1995).

The invention furthermore relates to a pharmaceutical composition, comprising one or more compounds effective as a selective opiate receptor modulator as defined above, and one or more compounds that are effective as an appetite depressant as defined above.

Pharmaceutical compositions according to the invention can be obtained or produced according to methods known in the art or analogously to these methods. Usually, the pharmaceutical compositions according to the invention are produced with non-chemical methods, for example by mixing the active ingredients, i. e. one or more modulating compounds (or a salts thereof) and/or one or more compounds that are effective as appetite depressant (or a salt thereof), and converting the mixture into the desired dosage form, for example into tablets by molding methods or into solutions by solving the active ingredients in a solvent. In general, the active ingredients are converted into a pharmaceutical composition together with one or more excipient, for example a solid, liquid and/or semiliquid excipient, or one or more auxiliaries and, if desired, in combination with one or more further active ingredients.

These preparations can be used as medicaments in human or veterinary medicine. Suitable excipients are organic or inorganic substances which are suitable for enteral (for example oral), parenteral or topical administration and do not react with the novel compounds, for example water, vegetable oils, benzyl alcohols, alkylene glycols, polyethylene glycols, glycerol triacetate, gelatine, carbohydrates, such as lactose or starch, magnesium stearate, talc or vaseline. Suitable for oral administration are, in particular, tablets, pills, coated tablets, capsules, powders, granules, syrups, juices or drops, suitable for rectal administration are suppositories, suitable for parenteral administration are

solutions, preferably oily or aqueous solutions, furthermore suspensions, emulsions or implants, and suitable for topical application are ointments, creams or powders. The novel compounds can also be lyophilized and the resultant lyophilizates used, for example, for the preparation of injection preparations. The preparations indicated may be sterilized and/or comprise assistants, such as lubricants, preservatives, stabilizers and/or wetting agents, emulsifiers, salts for modifying the osmotic pressure, buffer substances, dyes, flavours and/or a plurality of further active ingredients, for example one or more vitamins.

For administration as an inhalation spray, it is possible to use sprays in which the active ingredient is either dissolved or suspended in a propellant gas or propellant gas mixture (for example $CO_2$ or chlorofluorocarbons). The active ingredient is advantageously used here in micronized form, in which case one or more additional physiologically acceptable solvents may be present, for example ethanol. Inhalation solutions can be administered with the aid of conventional inhalers.

The modulating compounds according to the invention are generally administered in analogy to other known preparations available commercially for the indications claimed, preferably in doses of between about 0.001 mg and 50 mg, in particular between 0.01 and 30 mg, per dose unit. The daily dose is preferably between about 0.02 and 20 mg/kg, more preferred between about 0.05 and 10 mg/kg, even more preferred between about 0.1 and 5 mg/kg and in particular 0.2 and 4.0 mg/kg of body weight. In many cases, a daily dose of about 0.3 mg/kg, about 1.0 mg/kg, about 2.0 mg/kg, about 3.0 mg/kg or about 4.0 mg/kg and especially of about 0.3 mg/kg, about 1.0 mg/kg or about 3.0 mg/kg is advantageous. In many cases, it is advantageous if the daily dosis is given in two separate portions each comprising the half amount of the given daily dosis. In general, notes on the dosage of the modulating compounds in mg are based on the pharmaceutical effective compounds itself or, if the compound is administered as salt, for example as hydrochloride, on the weight of the compound as its salt. The dosage given in mg/kg is based on the body weight of the patient in kg to which the compound is administered.

The specific dose for each individual patient depends, however, on various factors, for example on the activity of the specific compound employed, on the age, body weight, general state of health and sex, on the diet, on the time and route of administration, and on the excretion rate, pharmaceutical combination and severity of the particular disorder to which the therapy applies. Oral administration is preferred.

For the administration of Asimadoline, the following dosages have proven beneficial:

- 0.1 to 2.0 mg/kg daily, preferably 0.3 to 1.5 mg/kg daily and especially 0.75 to 1.5 mg/kg daily, for example about 1.0 mg/kg daily; this dosages stand for a "lower dosis" according to the invention;
- 1.75 to 6.0 mg/kg daily, preferably 2.0 to 4.5 mg/kg daily and especially 2.5 to 3.5 mg/kg daily, for example about 3 mg/kg daily; this dosages stand for a "higher dosis" according to the invention.

Subject of treatment or administration according to the aspects of the invention is every patient in need of such a treatment or an administration, preferably an animal, especially and nonhuman mammalian, and especially preferred a human being.

Description of the figures:

Fig. 1 shows the results of the satiety test (maximum volume ingested in ml depending on the administered dosis of asimadoline for study Part A (three columns, from left to right: placebo → 280 ml; 0.15 mg/kg of asimadoline → 1425 ml; 0.5 mg/kg of asimadoline → 1470 ml) and for the study Part B (two columns, from left to right: placebo → 1300 ml; 1.5 mg/kg of asimadoline → 1390 ml).

Fig.2 shows the values (VAS scores) for the aggregate postprandial symptoms as the result of the ingested volume in the satiety test depending on the administered dosis of asimadoline for study Part A (three columns, from left to right: placebo → VAS-Score = 180; 0.15 mg/kg of asimadoline → VAS-Score = 187; 0.5 mg/kg of asimadoline → VAS-Score = 170) and for the study Part B (two columns, from left to right: placebo → VAS-Score =162; 1.5 mg/kg of asimadoline → VAS-Score = 192).

Fig. 3 shows the fasting volume (in ml) of the colon at 0 mm pressure as a result of the Barostat test depending depending on the administered dosis of asimadoline for study Part A (three columns, from left to right: placebo → 1 ml; 0.15 mg/kg of asimadoline → 8 ml; 0.5 mg/kg of asimadoline → 21 ml) and for the study Part B (two columns, from left to right: placebo → 6 ml; 1.5 mg/kg of asimadoline → 24 ml).

Fig. 4 shows the values (VAS scores) of the sensation to distension as a result of the Barostat test depending on

the pressure (mm Hg) that causes the distension and the administered dosis of asimadoline for studies Part A and Part B (4 groups, each comprised of five columns; from left to right:

- at 8 mm Hg
  - placebo (Part A) → 37;
  - 0.15 mg/kg of asimadoline (Part A) → 38;
  - 0.5 mg/kg of asimadoline (Part A) → 26
  - placebo (Part B) → 20;
  - 1.5 mg/kg of asimadoline (Part B) → 31;
- at 16 mm Hg
  - placebo (Part A) → 43;
  - 0.15 mg/kg of asimadoline (Part A) → 37;
  - 0.5 mg/kg of asimadoline (Part A) → 37;
  - placebo (Part B) → 23;
  - 1.5 mg/kg of asimadoline (Part B) → 38;
- at 24 mm Hg
  - placebo (Part A) → 43;
  - 0.15 mg/kg of asimadoline (Part A) → 45;
  - 0.5 mg/kg of asimadoline (Part A) → 41;
  - placebo (Part B) → 42;
  - 1.5 mg/kg of asimadoline (Part B) → 41;
- at 32 mm Hg
  - placebo (Part A) → 54;
  - 0.15 mg/kg of asimadoline (Part A) → 53;
  - 0.5 mg/kg of asimadoline (Part A) → 47)
  - placebo (Part B) → 51;
  - 1.5 mg/kg of asimadoline (Part B) → 43;

Fig. 5 shows the values (VAS scores) of the pain to distension as a result of the Barostat test depending on the pressure (mm Hg) that causes the distension and the administered dosis of asimadoline for studies Part A and Part B (4 groups, each comprised of five columns; from left to right:

- at 8 mm Hg
  - placebo (Part A) → 22;
  - 0.15 mg/kg of asimadoline (Part A) → 25;
  - 0.5 mg/kg of asimadoline (Part A) → 18
  - placebo (Part B) → 14;
  - 1.5 mg/kg of asimadoline (Part B) → 30;
- at 16 mm Hg
  - placebo (Part A) → 33;
  - 0.15 mg/kg of asimadoline (Part A) → 28;
  - 0.5 mg/kg of asimadoline (Part A) → 28;
  - placebo (Part B) → 21;
  - 1.5 mg/kg of asimadoline (Part B) → 37;
- at 24 mm Hg
  - placebo (Part A) → 38;

    - 0.15 mg/kg of asimadoline (Part A) → 30;
    - 0.5 mg/kg of asimadoline (Part A) → 32;
    - placebo (Part B) → 30;
    - 1.5 mg/kg of asimadoline (Part B) → 40;

- at 32 mm Hg

    - placebo (Part A) → 48;
    - 0.15 mg/kg of Asimadoline (Part A) → 42;
    - 0.5 mg/kg of asimadoline (Part A) → 38)
    - placebo (Part B) → 43;
    - 1.5 mg/kg of asimadoline (Part B) → 47;

**List of abbreviations used in the text**

| | |
|---|---|
| AC | ascending colon |
| AE | Adverse event |
| ALT | Alanine aminotransferase |
| ANCOVA | Analysis of Covariance (statistical method) |
| ANOVA | Analysis of Variance Applet (statistical method) |
| a.m. | ante meridiem; in the morning |
| AST | Aspartate aminotransferase |
| $AUC_{0-t}$ | area under the concentration time curve from time zero to time t |
| $AUC_{0-\infty}$ | total area under the concentration time curve |
| $AUC\tau$ | area under the concentration time curve at steady state |
| b.i.d. | bis in die; twice daily |
| BMI | Body mass index |
| C | Celsius |
| $C_{av}$ | average plasma concentration |
| cc | Cubic centimeter |
| CF | Colonic filling |
| CL/f | apparent total body clearance of drug from plasma |
| Cmax | Concentration maximum |
| COX2 | Cyclo-oxygenase 2 |
| CNS | Central nervous system |
| CPMP | Committee for Proprietary Medicinal Products |
| $C_{pre}$ | trough plasma concentrations |
| CRDO | Clinical Research & Development Organization |
| CRF | Case report form |
| CV | Coefficient of variance |
| CYP | Cytochrome P |
| DC | descending colon |
| dl | decilitre |
| ECG | Electrocardiogram |
| EMD | Substance code of Merck KGaA, Darmstadt, Germany |
| EMR | Study code of Merck KGaA, Darmstadt, Germany (sponsor of the clinical study) |
| F | Fahrenheit |
| FDA | Food and Drug Administration |
| g | Gram |
| G | accelerative force |
| GC | geometric center |
| GCP | Good Clinical Practice |
| GE | gastric emptying |
| GI | gastrointestinal |
| GMP | Good Manufacturing Practice |
| HADS | Hospital Anxiety and Depression Scale |

| | |
|---|---|
| hrs | hours |
| IBS | Irritable Bowel Syndrome |
| $IC_{50}$ | Inhibiting concentration at 50 % |
| ICH | International Conference on Harmonisation |
| IND | Investigational Exemption of a New Drug |
| IRB | Institutional Review Board |
| κ | kappa |
| kcal | calorie |
| kg | Kilogram |
| KGaA | Kommanditgesellschaft auf Aktien |
| L | Litre |
| LC-MS | liquid chromatography mass spectrometry |
| μmol | Micromole |
| μmol/l | Micromole per litre |
| mCi | MicroCurie |
| mEq/l | MilliEquivalent per litre |
| mg | Milligram |
| mg/dl | Milligram per decilitre |
| mg/kg | Milligram per kilogram |
| mg/ml | Milligram per millilitre |
| min | minute |
| ml | Millilitre |
| ml/min | Millilitre per minute |
| mm | Millimetre |
| mmHg | Millimetre mercurv |
| mRNA | magnetic resonance |
| μ | micro |
| N | number |
| no. | number |
| NSAID | non-steroidal anti-inflammatory drugs |
| PET | Positron emission tomography |
| pH | Potential of hydrogen |
| p.m. | post meridiem; in the afternoon/evening |
| PMX-CTM | computer program for randomization |
| QTc | Corrected QT interval |
| RS | rectosigmoid colon |
| δ | delta |
| SAE | Serious adverse event |
| SAS | Statistical Analysis SystemTM registered trademark of SAS Institute, Inc. |
| TAT | Therapeutic Area Team |
| TC | transverse colon |
| $t_{max}$ | time to reach the maximal plasma concentration |
| $T_{1/2}$ | apparent elimination half-life |
| U/l | Units per litre |
| UK | United Kingdom |
| VAS | Visual Analog Scales |
| $V_z/f$ | apparent volume of distribution during terminal phase |

**Examples**

A a single center, randomized, double-blind, placebo controlled, parallel group Phase I study has been performed. The trial evaluates the effects of a 7-day treatment with 2 different doses of the peripherally selective opiate receptor modulator asimadoline (N-methyl-N-[(1S)-1-phenyl-2-((3S)-3-hydroxypyrrolidin-1-yl)ethyl]-2,2-diphenylacetamide hydrochloride, EMD 61 753) on gastrointestinal and colonic transit and sensorimotor functions in healthy subjects in comparison to placebo.
Specific aims of the study are to compare gastrointestinal and colonic transit profiles and the effects on:

1. colonic aggregate sensation score in response to distension

2. thresholds for colonic first sensation and sensation of pain in response to distension
3. fasting colonic compliance and tone, and
4. postprandial tonic response to standard meal ingestion.

The study included 60 healthy subjects with no history of gastrointestinal symptoms, particularly no evidence of irritable bowel syndrome have been randomized into one of the following treatment groups:

| | | |
|---|---|---|
| 1. | Asimadoline 0.15 mg b.i.d. for 7 days | 20 subjects |
| 2. | Asimadoline 0.5 mg b.i.d. for 7 days | 20 subjects |
| 3. | Placebo b.i.d. for 7 days | 20 subjects |

The randomization assignments to ensure a balance on age and gender in the treatment groups has been made to according to standard procedures. Subjects (and the primary investigators) have been blinded to treatment assignment and the treatment groups have been balanced on age and gender.

All subjects have undergone a satiety test prior to and under medication, a scintigraphic gastric and colonic transit test, and, after overnight bowel preparation, a barostat test assessing colonic compliance and colonic sensation prior to and under medication, and fasting colonic tone and colonic response to a standardized meal under medication.

The primary sensory endpoints in the study are the pain, gas, and aggregate (average of pain and gas) sensation score at four (randomly ordered) phasic distensions of the colon (8, 16, 24, and 32 mmHg). The primary motor endpoints in the study are gastric emptying (% remaining in the stomach at 2 hrs), colonic filling (%) at 6 hrs, colonic geometric center (GC) at 24 hrs, and maximum satiety volumes.

Secondary analysis variables include thresholds for colonic sensation of gas and pain, overall gas score, overall pain score, and overall aggregate score, colonic compliance, fasting colonic tone, colonic tone response to standard meal ingestion, colonic transit summarized by GC at 4 and 48 hours, and percent remaining in the stomach at 4 hrs. In the satiety test, the individual symptom scores (bloating, fullness, nausea, pain) have been described. The safety assessment has included recording of adverse events (AEs). Subjects have also undergone a complete physical examination, ECG recording, and have provided blood and urine specimens for routine laboratory safety tests. In addition, quantitative determination of asimadoline in plasma has been performed.

Specific aims of this study have been the comparison of the effects of 7 day treatment with placebo and three doses of asimadoline as described above on:

- satiety after ingestion of a nutrient drink
- colonic aggregate sensation score in response to distension in healthy subjects
- thresholds for colonic sensation of gas and pain in response to distension in healthy subjects
- fasting colonic compliance and tone
- postprandial tonic response to standard meal ingestion

A further aim of the study was to compare the gastrointestinal and colonic transit profiles during the 7-day treatment. The studies on lower dosage (0.15 mg/kg and 0.5 mg/kg versus placebo) were performed separately as **Part A** and the studies on higher dosage (1.5 mg/kg versus placebo) were performed separately as **Part B** of the studies (see figures 1 to 4).

The following results were obtained:

a) satiety test (see figures 1 and 2)

- 0.15 mg/kg asimadoline increase the maximum ingested volume slightly versus placebo;
- 0.5 mg/kg asimadoline increase the maximum ingested volume significantly versus placebo;
- dose response effect established at the lower end of the dosis range (0.15 mg/kg);
- ceiling effect established at higher concentration (1.5 mg/kg);
- higher ingested volume was not associated with an increase in symptoms at a dosis in the range of 0.5 mg/kg;
- at a dosis of 1.5 mg/kg symptoms increased with increased volume ingested.

b) Barostat test (see figures 3 to 5)

- the fasting volume is significantly higher at a dosis of 0.5 mg/kg versus placebo;
- at a dosis of 0.5 mg/kg, the perception of distension is significantly reduced at a low pressure (8 mm Hg); this effect decreases by increasing pressure;

- no significant reduction in the perception of pain could be observed;
- at higher doses (1.5 mg/kg) significantly higher scores for pain and sensation were observed versus placebo.

The results show clearly that asimadoline is suitable for the dosis dependent regulation of appetite. At lower doses, it promotes an increased uptake of food and increases the volume of food ingested without affecting negative postprandial symptoms, i. e. without increasing bloating, fullness, nausea and/or pain.

Intubated Colonic Procedures (Barostat test)

*Bowel preparation*

All subjects are presented to the General Clinical Research Center at Charlton 7, General Clinical Research Center, on visit 3 after overnight bowel preparation with the oral colonic lavage solution (2-5 L of polyethylene glycol 3350 and electrolyte solution, NuLytely™, Abbott Laboratories, Chicago. IL), and a 12-hour fast.

*Tube placement*

Flexible colonoscopy is performed to evaluate the left side of the colon and to place a teflon guidewire into the proximal colon under fluoroscopic control. The endoscope is withdrawn.

A barostat catheter is inserted into the colon along the guidewire so that the barostat balloon is located in the upper sigmoid or descending colon. The catheter is connected to a barostat machine using an infinitely compliant 10-cm long balloon with a maximum volume of 600 cc (Hefty Baggies, Mobil Chemical Co., Pittsford, NY) linked to an electronic barostat (Mayo rigid barostat, Mayo Foundation Engineering Department, Rochester, MN) which has a rigid piston. The manometric portion comprises six waterperfused (0.4 ml/min) pneumohydraulic sensors, three in the descending colon (sensor numbers 1-3) and three in the sigmoid colon (sensor numbers 4-6). The manometric sensors are 5 cm apart, while the first and second sensors are 5 cm oral and caudal to the balloon respectively. To decrease the effects of abdominal viscera on the balloon volume, the studies are performed with the subjects in a semi-prone position during the entire duration of the study.

*Colonic compliance and sensation*

Previous studies have shown that an initial "conditioning" distension to 20 mmHg renders subsequent assessments of compliance and perception more reproducible [15. 47. 48]. Following the conditioning distension, colonic compliance and sensory thresholds are measured by ramp inflation with increments of 4 mmHg, o steps at 30 second intervals from 0 to 44 mmHg; thresholds for first sensation and sensation of pain are thus determined using the ascending method of limits.

Immediately prior to assessment of colonic sensation, four 100 mm visual analog scales (VAS) scales using the anchor points "tired-energetic", "peaceful-tense" and "worried-relaxed" and "active-drowsy" areused to determine the level of arousal, anxiety or stress being experienced by the subject. This has previously been shown to be a significant covariate in the assessment of visceral sensation scores. Subsequently, randomized-order phasic distensions at 8, 16, 24, and 32 mm Hg above operating pressure areapplied to measure the sensations of gas or pain.

For rating sensory perception, the participants areasked to mark two separate VAS for abdominal pain and feeling of gas at a standardized time, 20 seconds after the distension had commenced. The VAS are anchored at the ends by the descriptions "unnoticeable" and "unbearable". During assessment of sensation, verbal interaction between the subject and investigator is minimized.

*Repeated Measurement of Colonic Sensation*

Colonic sensation is assessed before and 1 h after drug administration during the measurement of colonic compliance. This is equivalent to finding the threshold pressures or the initial perception and pain perception during sequential pressure increments using the ascending method of limits. This approach has been shown to provide an assessment of thresholds which is as accurate as tracking with or without random staircase method [49].

*Colonic response to a standard meal*

The participant is allowed to rest for 15 minutes. Then, fasting colonic tone and phasic activity is recorded for 30 minutes. Colonic tone is assessed by noting the changes in the balloon volume in the presence of a constant operating pressure in the balloon. After transient inflation of the barostat bag to a volume of 75 ml to ensure the unfolding of the

bag, it is deflated and inflated with 1 mmHg increments of pressure. The operating pressure is defined as 2 mmHg above the minimal distension pressure at which respiratory excursions are clearly recorded from the barostat tracing, or when respiratory variations are not obvious, that is, the pressure at which the volume of the bag is 25 ml.

Assessment of fasting colonic tone is followed by a 90 minute measurement of colonic tone after consuming a chocolate milkshake containing 1.000 kcal (35 % carbohydrate. 53 % fat and 12 % protein). This standard liquid high fat meal is administered to induce the colonic response to feeding.

When the recording is finished, the assembly is removed by gentle traction of the tube.

*Measurements to he subjected to data analysis*

- colonic compliance is measured by ramp inflation using 4 mmHg increments of pressure every 30 seconds, prior to and under medication
- threshold for first sensation and sensation of pain prior to and under medication
- pain and gas and aggregate symptom scores over the four phasic distension levels
- prior to and under medication
- fasting colon tone (ml) only under medication
- postprandial change in colonic tone only under medication

*Scintigraphic transit test [42-45]*

*Procedure*

Subjects arrive at Gastroenterology Research Unit, fasted, at 7:00 a.m. on visit 5. Results of the pregnancy test performed on the previous day are reviewed, and the study drug is administered as well as the $^{111}InCl_3$ capsule. Typically one hour later, a breakfast $^{99m}Tc$ test meal is administered and gamma camera images are obtained for several hours (see below) after test meal ingestion. The subject leaves the study center at the end of the afternoon. He/she is asked to return the following 2 days, visit 6, and visit 7, for further images.

*Gastric emptying transit*

Subjects are studied on visit 5 following an overnight fast. One (1.0) mCi $^{99m}Tc$ Sulfur colloid is added to two raw eggs during the scrambling, cooking process. The eggs are served on one slice of buttered bread along with one 8-ounce glass of 1 milk (total calories: 296 kcal, 32 % protein, 35 % fat. 33 % carbohydrate). Anterior and posterior gamma camera images are obtained at 0, 1, 2, 3, 4, and 6 hours after meal ingestion on visit 5.

*Image schedule for gastric emptying*

| | $^{99m}Tc$ meal | Images | | | | | |
|---|---|---|---|---|---|---|---|
| Intervals (h) | | 0 | 1 | 2 | 3 | 4 | 6 |
| Time | 8:00 | 8:00 | 9:00 | 10:00 | 11:00 | 12:00 | 14:00 |

*Colonic transit test*

$^{111}InCl_3$ (0.10 mCi) is mixed with a slurry of 5 mg activated charcoal. The slurry is evaporated to dryness on a hot plate at 90 °C, and the dried charcoal is placed into a size one gelatine capsule (Eli Lilly, Indianapolis, IN) and coated with methacrylate (Eudragit S100) as in previous studies [43. 45, 46]. A marker, to be used to map the location of the capsule, is placed on the subject's anterior superior iliac spine. The capsule is administered with a 3-ounce glass of

water. Once imaging confirms the capsule has been emptied from the stomach (observed by the position of capsule relative to iliac crest markers), the radio labeled egg meal is administered. This typically occurs within one hour; rarely, capsule does not empty. In these circumstances, the meal is administered anyway after one hour because of the timing of administration of the study drug, and the need to assess accurately the effect of study drug on gastric and small bowel transit. Anterior and posterior gamma camera images are obtained 4, 6, 8, 24, 32 and 48 hours after ingestion of the $^{111}InCl_3$ capsule on visit 5, 6, and 7.

A standardized meal (550 kcal. chicken, potato and pudding) is given 4 hours after ingestion of the radio labeled meal. All other meals are ingested ad libitum.

*Image schedule for colonic transit*

| | $^{111}InCl_3$ | Images | | | | | |
|---|---|---|---|---|---|---|---|
| Intervals (h) | | 4 | 6 | 8 | 24 | 32 | 48 |
| Time | 7:00 | 11:00 | 13:00 | 15:00 | 7:00 | 15:00 | 7:00 |

Data analysis

Data is analyzed as described in previous studies [15, 16,43,45]

Geometric mean of counts in anterior and posterior gastric regions of interest are used to estimate the proportion of $^{99m}Tc$ emptied at 2 and 3 hours (gastric emptying). The proportion of $^{99m}Tc$ reaching the colon at 6 hours is also estimated as a measure of orocaecal transit (a surrogate for small bowel transit).

Geometric center at 4, 24, 32 and 48 hours is estimated using geometric mean of counts in ascending, transverse, descending and rectosigmoid colon and stool (weighted by factors of I to 5 respectively). The primary variable of interest is the geometric center at 34 hours.

The geometric center is the weighted average of counts in the different colonic regions [ascending (AC), transverse (TC), descending (DC), rectosigmoid (RS)] and stool. At any time, the proportion of colonic counts in each colonic region is multiplied by its weighting factor as follows:

$$(\%AC \times 1 + \%TC \times 2 + \%DC \times 3 + \%RS \times 4 + \%\ stool \times 5)/100 = \text{geometric center}$$

Thus, a high geometric center implies faster colonic transit; for example, a geometric center of 1 implies all isotope is in the ascending colon and a geometric center of 5 implies all isotope is in the stool.

*Statistical methodology and analysis*

*Primary and secondary target variables*

The primary SENSORY endpoints are the actual values of pain, gas sensation or aggregate sensation VAS under the individual barostat pressures of 8, 16, 24, and 32 mmHg.

The primary MOTOR endpoints are the gastric emptying at 2 h, colonic filling, at 6 h. colonic geometric center of transit at 24 h and the colonic tone response to standardized meal ingestion.

The primary endpoint in the satiety test is difference to baseline in the aggregate satiety score 30 min after full satiety.

Secondary endpoints:

- Colonic compliance

- Thresholds for colonic sensation of gas and pain in response to distension
- Difference to baseline in thresholds for colonic sensation of gas and pain in response
- to distension
- Values of pain, gas sensation or aggregate sensation VAS as average over all individual barostat pressures of 8, 16, 24, and 32 mmHg.
- Fasting colonic tone
- Colonic tone response to standard meal ingestion
- Colonic transit summarized by GC at 4 and 48 hours;
- % emptied from stomach at 4 hours;
- Difference to baseline in nutrient drink volume ingested at full satiety
- Difference to baseline in the individual symptom scores (bloating, fullness, nausea, pain) 30 min after full satiety.

All efficacy endpoints are computed by the Mayo Clinic study statistician from the raw data recorded.

Pharmacokinetic assessments:

The pharmacokinetic parameters determined from the concentration-time data of asimadoline are:

$C_{max}$, $C_{pre}$, $t_{max}$ and $AUC_{0-t}$

Descriptive statistics are performed on these parameters by the Department of Clinical Pharmacology of Merck KGaA.

**Definitions of evaluability**

Safety

The safety population includes all randomized subjects who have taken at least one dose of active treatment.

Intention-to-treat

The intention-to-treat population includes all randomized subjects who have taken at least one dose of active treatment and who provide any follow-up data for one or more efficacy target variables.

**Per protocol**

The per protocol population includes all subjects who have been treated according to protocol and fulfil the following criteria:

- All inclusion/exclusion criteria satisfied, unless some criteria were waived
- Absence of relevant protocol violations with respect to factors likely to affect the efficacy of treatment
- Adequate study medication compliance
- Measurements of most (> 90 %) primary target variables at all visits

**Description of statistical analysis**

The primary goal of this study is to compare the responses (colonic sensation, gastric emptying (GE), and colonic transit) among the three treatment groups (placebo, 0.15 mg and 0.5 mg). The treatment assignments remain blinded to the primary investigator(s) until all response data are edited and documented in a SAS™ database developed in the Section of Biostatistics at the Mayo Clinic.

The primary analyses of treatment effects include all randomized subjects based on the intent-to-treat principle. Randomized subjects with missing data are assigned an appropriate 'treatment failure' value for these analyses. Additional analyses and summaries of the response data (by treatment group) focus on those subjects with complete data and adequate study medication compliance (per protocol). A descriptive summary of subject characteristics (e.g., age, gender, body mass index (BMI), satiety test) at baseline is compiled overall subjects randomized and by treatment group.

The assessment of colonic sensation (gas, pain, and the aggregate [average of gas and pain]scores) is based on a repeated measures analysis of covariance. An unstructured variance-covariance matrix for the four repeated values (score at 8, 16, 24, and 32 mmHg) is used if a compound-symmetry structure is unwarranted. This analysis is done separately for gas, pain, and the aggregate scores; no adjustment in the alpha level (0.05) for multiple types of response

endpoints (different scores) is done. The potential covariates in this analysis include age, gender, body mass index, predrug sensation scores, corresponding distending volumes, and level of anxiety and tension recorded on the day of assessment.

The analysis of primary motility endpoints (gastric residual percent at 2 hrs, colonic filling (CF) percent at 6 hrs. and colonic geometric center at 24 hrs)and the maximum satiety volume (at day 5) is based on one-way analysis of variance or analysis of covariance methods. The proportions (GE at 2 hrs and CF at 6 hrs)may warrant transformation (e.g., $\sin^{-1}\sqrt{}$) prior to analysis to stabilize variation across treatment groups. Analysis of the satiety volumes incorporates the baseline value as a covariate in addition to BMI; or alternatively, the relative changes (log [day 5 volume/baseline volume]) are analyzed with BMI as a covariate.

The analysis of secondary response variables (colonic compliance. fasting colonic tone [i.e., volume], relative change in colonic volume in response to ingestion of a standard meal, relative changes in thresholds for colonic sensation of gas and pain, GE at 4 hrs, and the GC values at 4 and 48 hrs) are also based on one-way analysis of variance or covariance methods, employing appropriate transformations as necessary.

For both the primary and secondary analyses, simple non-parametric (Kruskal-Wallis test) comparisons among the three groups are also examined to complement the previously described analyses. The differences in mean response values between treatment groups are estimated via 95 % confidence intervals using the (pooled) estimate of variation from the analysis of variance or covariance results, unless substantial heterogeneity of variance is indicated. All statistical tests use a two-sided alpha level of 0.05. No adjustment in alpha level for multiple (types of) endpoints is done, though multiple (pairwise) comparisons between treatment groups for any one given endpoint are made at an alpha level of 0.017 (i.e., Bonferroni adjustment for three pairwise comparisons). In addition, 95 % confidence intervals for the differences in group means also are computed and reported to provide unadjusted pairwise comparisons.

The efficacy analysis is the responsibility of the study statistician Alan Zinsmeister in the section of Biostatistics Mayo Clinic Rochester.

A summary of incidence, type, and severity of adverse events, relevant laboratory values, and other safety-related data is compiled by Merck KGaA, Darmstadt, Department Corporate Biometrics.

**Sample size**

The proposed sample size (N =20 per treatment group) provides 80 % (90 %) power to detect the effect sizes listed below between two groups based on a simple two-sample t-test. The analysis of variance (or covariance) provides similar power for somewhat smaller (overall) differences depending on their pattern.

| Response | CV+ (%) | Effect size (%)*detectable with: | |
|---|---|---|---|
| | | 80% power | 90 % power |
| GE @ 2 hrs | 43 % | 38 % | 44 % |
| GE @ 24 hrs | 38 % | 34 % | 39 % |
| CF @ 6 hrs | 51 % | 45 % | 52% |
| Satiety Volume | 25 % | 22 % | 26 % |
| Fasting Colonic Tone | 41 % | 36 % | 42 % |
| Colonic Meal Responses | 43 % | 38 % | 44 % |
| GC @ 4 hrs | 65% | 58 % | 67% |

The estimates of effect size from results of previous studies for the individual barostat pressures and overall gas, pain, and aggregate score are given in the table below corresponding to 80 % and 90 % power for N=20 vs N=20 and for N=20 vs N=40 (e.g., placebo vs overall drug):

| Response | CV (%)[2] | Effect size (%)[1] detectable with: | | | |
|---|---|---|---|---|---|
| | | 80 % power | | 90 % power | |
| | | N = 20 | N = 40[3] | N = 20 | N = 40[3] |
| Gas 8 mmHg | 103 % | 91 % | 79 % | 106% | 91 % |
| Gas 16 mmHg | 80 % | 71 % | 61 % | 82 % | 71 % |

(continued)

| Response | CV (%)[2] | Effect size (%)[1] detectable with: | | | |
|---|---|---|---|---|---|
| | | 80 % power | | 90 % power | |
| | | N = 20 | N = 40[3] | N = 20 | N = 40[3] |
| Gas 24 mmHg | 80 % | 71 % | 61 % | 82 % | 71 % |
| Gas 32 mmHg | 75 % | 66 % | 58 % | 77 % | 67 % |
| Pain 8 mmHg | 92 % | 82 % | 71 % | 94 % | 82 % |
| Pain 16 mmHg | 90 % | 80 % | 69 % | 92 % | 80 % |
| Pain 24 mmHg | 78 % | 69 % | 60 % | 80 % | 69 % |
| Overall Gas Score[4] | 73 % | 65 % | 56 % | 75 % | 65 % |
| Overall Pain Score[4] | 71 % | 63 % | 54 % | 73 % | 63 % |
| Overall Aggregate Score[4] | 61 % | 54 % | 47 % | 63 % | 54 % |

[1] Difference between groups as a percentage of overall mean
[2] Coefficient of variance
[3] Analysis of N=20 (placebo) vs. N=40 (overall drug)
[4] Average value over 8, 16, 24, and 32 mmHg

To reduce variability in this study, the treatment groups are balanced on age (age between 50 and 60 years) and gender prior to inclusion in the study.

Further embodiments

Also disclosed are:

1. The use of a compound that is effective as selective opiate receptor modulator for the manufacture of a pharmaceutical for diagnosis and/or the treatment of disorders, said disorders being selected from eating disorders and digestive disorders.

2. The use according to embodiment 1, **characterized in that** said receptor modulator is a receptor agonist.

3. The use according to embodiment 1 or 2, **characterized in that** said receptor modulator is peripherally selective to the receptor.

4. The use according to one of the embodiments 1, 2 or 3, **characterized in that** said receptor is a kappa-opiate receptor.

5. The use according to one of the preceding embodiments, **characterized in that** the compound is selected from group consisting of Alvimopan, Loperamide, Asimadoline, Fedotozine, Pentazocine, U62066E, ICI204448, U-50488H, ADL 10-0101, ADL 10-0116 and ADL 1-0398.

6. The use according to one of the preceding embodiments, **characterized in that** the disorders are selected from the group consisting of regulation of pathological imbalanced appetite, cachexy, anorexia, dysorexia, dysponderosis, adiposity, bulimia, obesity, gastroparesis, gastroatonia, gastroparalysis and stenosis of the gastrointestinal tract.

7. The use of compound as defined in one of the embodiments 1 to 5, for the manufacture of a pharmaceutical effective for modulating the gastrointestinal tonus.

8. The use of compound as defined in one of the embodiments 1 to 5, for the manufacture of a pharmaceutical to be used in combination with one or more pharmaceuticals that are effective as an appetite depressant.

9. The use according to embodiment 8, **characterized in that** the pharmaceutical that is effective as an appetite

depressant is a sympathomimeticum.

10. The use according to embodiment 8 or 9, **characterized in that** the pharmaceutical that is effective as an appetite depressant is selected from the group consisting of Phenylpropanolamin, Cathin, Sibutramin, Amfepramon, Ephedrin and Norpseudoephedrin.

11. A pharmaceutical composition, comprising one or more compounds effective as a selective opiate receptor modulator as defined in one of the embodiments 1 to 5, and one or more compounds that are effective as an appetite depressant.

12. The pharmaceutical composition according to embodiment 11, **characterized in that** at least one of the compounds that are effective as a selective opiate receptor modulators is selected from group consisting of Alvimopan, Loperamide, Asimadoline, Fedotazine, ADL 10-0116 and ADL 1-0398.

13. The pharmaceutical composition according to embodiment 11 or 12, **characterized in that** at least one of the compounds that are effective as an appetite depressant is selected from group consisting of Phenylpropanolamin, Cathin, Sibutramin, Amfepramon, Ephedrin and Norpseudoephedrin.

14. The use of a pharmaceutical composition according to one of the embodiments 11, 12 or 13 for the treatment of diseases, said diseases being selected from the group consisting of regulation of pathological imbalanced appetite, cachexy, anorexia, dysorexia, dysponderosis, adiposity, bulimia, obesity, gastroparesis, gastroatonia, gastroparalysis and stenosis of the gastrointestinal tract.

15. A method for manufacture of a pharmaceutical composition according to one of the embodiments 11 to 13, **characterized in that** one or more compounds effective as selective opiate receptor modulator as defined in one of the embodiments 1 to 5, one or more compounds effective as appetite depressant as defined in one of the embodiments 8 to 10 and, optionally, one or more excipient and/or one or more auxiliaries are mixed together and converted into a pharmaceutical composition suitable for administration.

16. A pharmaceutical composition, **characterized in that** it comprises a therapeutic effective amount of at least one compound effective as selective opiate receptor modulator as defined in one of the embodiments 1 to 5 and at least one compound effective as appetite depressant as defined in one of the embodiments 8 to 10.

17. A set comprising separate packs of

(a) one or more compounds effective as a selective opiate receptor modulator as defined in one of the embodiments 1 to 5 and/or a salt and/or a solvate thereof
and
(b) one or more compounds effective as appetite depressant as defined in one of the embodiments 8 to 10 and/or a salt and/or a solvate thereof.

18. A method of treatment of obesity characterized in administering one or more selective opiate receptor modulators in high doses to a patient in need of such a treatment.

19. The method of treatment according to embodiment 18, **characterized in that** the dosis ranges from about 2.0 mg/kg daily to about 10 mg/kg daily.

20. A method of treatment of anexoria characterized in administering one or more selective opiate receptor modulators in lower doses to a patient in need of such a treatment.

21. The method of treatment according to embodiment 20, **characterized in that** the dosis ranges from about 0.1 mg/kg daily to about 1.9 mg/kg daily.

## Claims

**1.** N-methyl-N-[(1S)-1-pheny)-2-((3S)-3 hydroxypyrrolidin-1-yl)ethyl]-2,2-diphenylacetamide or a pharmaceutically acceptable salt thereof for use as a medicament for treating a digestive disorder, whereby the compound is effective

for modulating the gastrointestinal tonus.

**2.** The compound according to claim 1, whereby the compound is effective for modulating one or more postprandial symptoms.

**3.** The compound according to claim 2, wherein the postprandial symptoms are selected from the group consisting of bloating, fullness, nausea, and pain following ingestion of food.

**4.** The compound according to claim 2, wherein the postprandial symptom is pain following ingestion of food.

**5.** The compound according to claim 1, whereby the compound is effective for modulating satiety.

**6.** The compound according to claim 1, whereby the digestive disorder is selected from the group consisting of cachexy, gastroparesis, gastroatonia, gastroparalysis and stenosis of the gastrointestinal tract.

**7.** The compound according to claim 1, wherein the pharmaceutically acceptable salt is N-methyl-N-[(1S)-1-phenyl-2-((3S)-3-hydroxypyrrolidin-1-yl)ethyl]-2,2-diphenylacetamide hydrochloride.

**8.** The compound according to claim 7, whereby the compound is effective for modulating one or more postprandial symptoms.

**9.** The compound according to claim 8, wherein the postprandial symptoms are selected from the group consisting of bloating, fullness, nausea, and pain following ingestion of food.

**10.** The compound according to claim 8, wherein the postprandial symptom is pain following ingestion of food.

Figure 1

Maximum volume ingested / ml (higher is better)

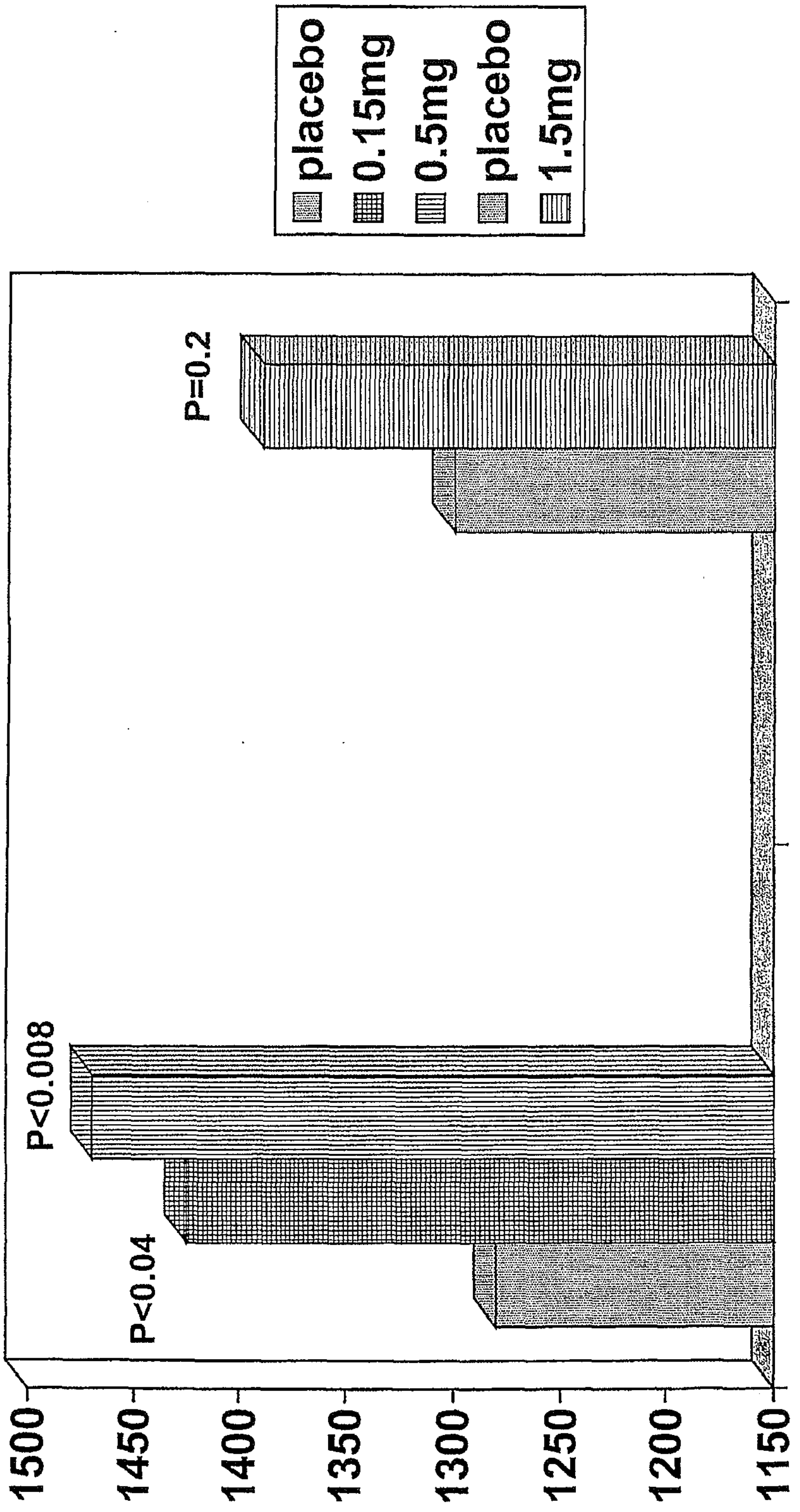

Figure 2

VAS score (lower is better)

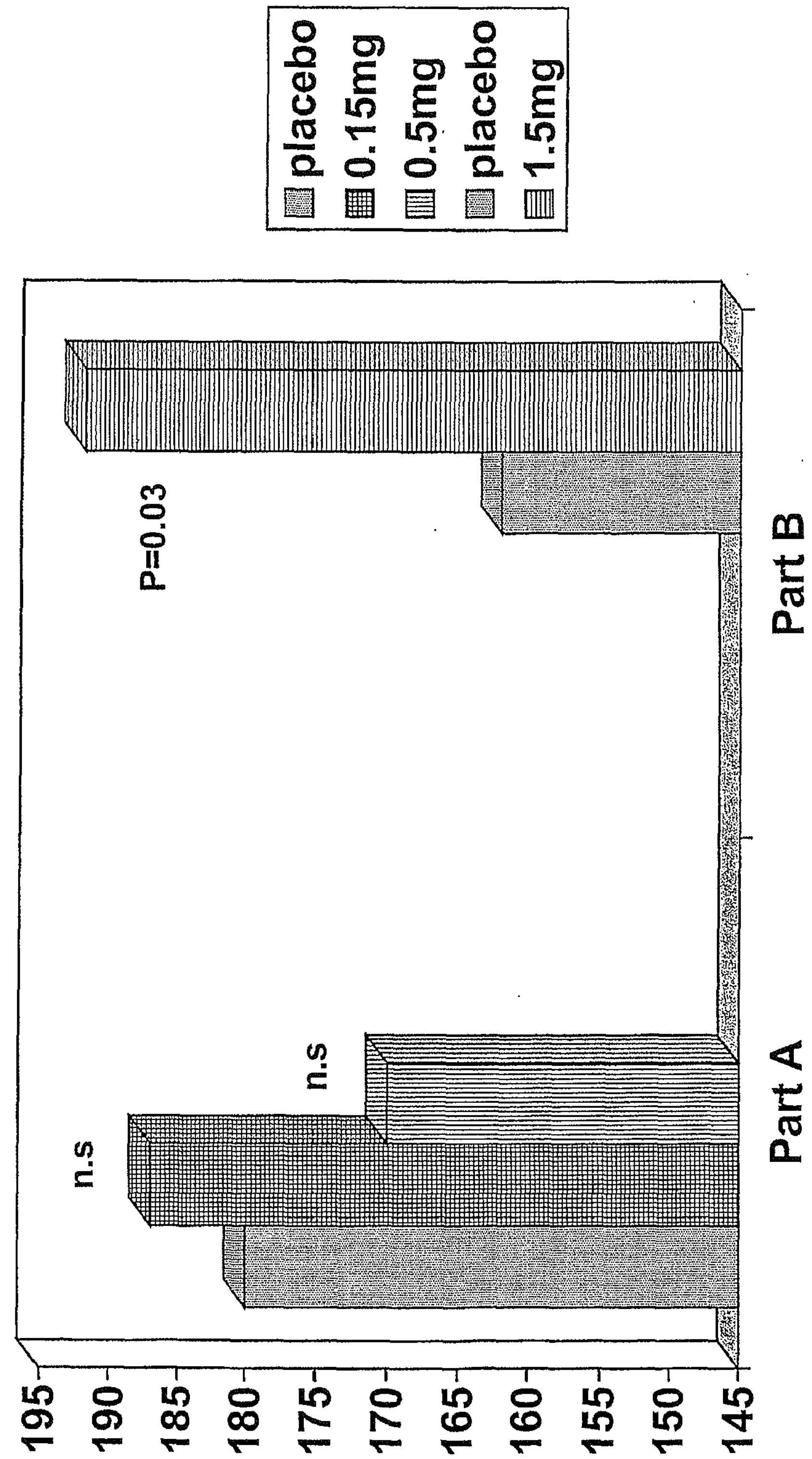

Figure 3

Colonic volume at 0 pressure / ml (higher indicates relaxation)

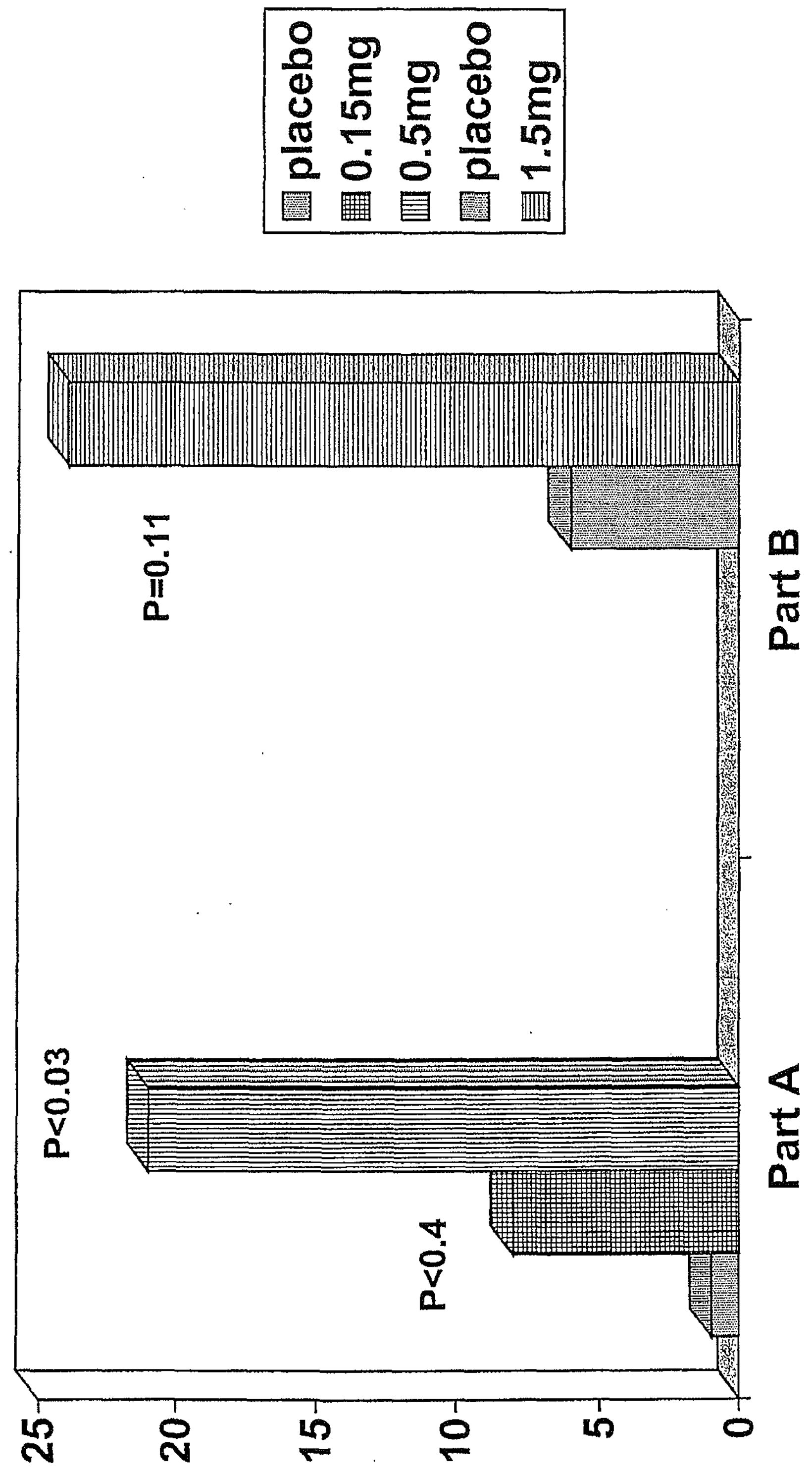

Figure 4

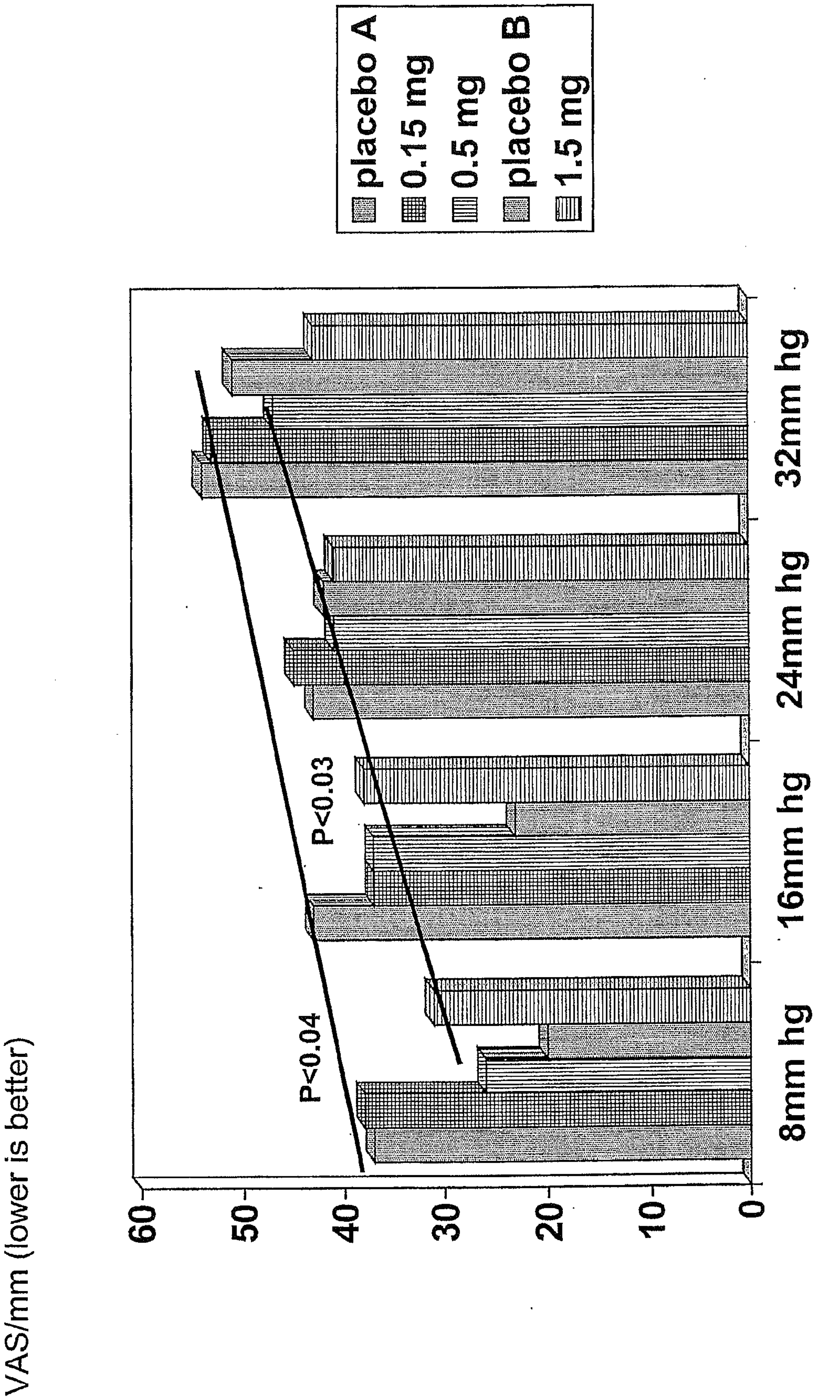
VAS/mm (lower is better)
60
50
40
30
20
10
0
P<0.04
P<0.03
8mm hg
16mm hg
24mm hg
32mm hg
placebo A
0.15 mg
0.5 mg
placebo B
1.5 mg

Figure 5

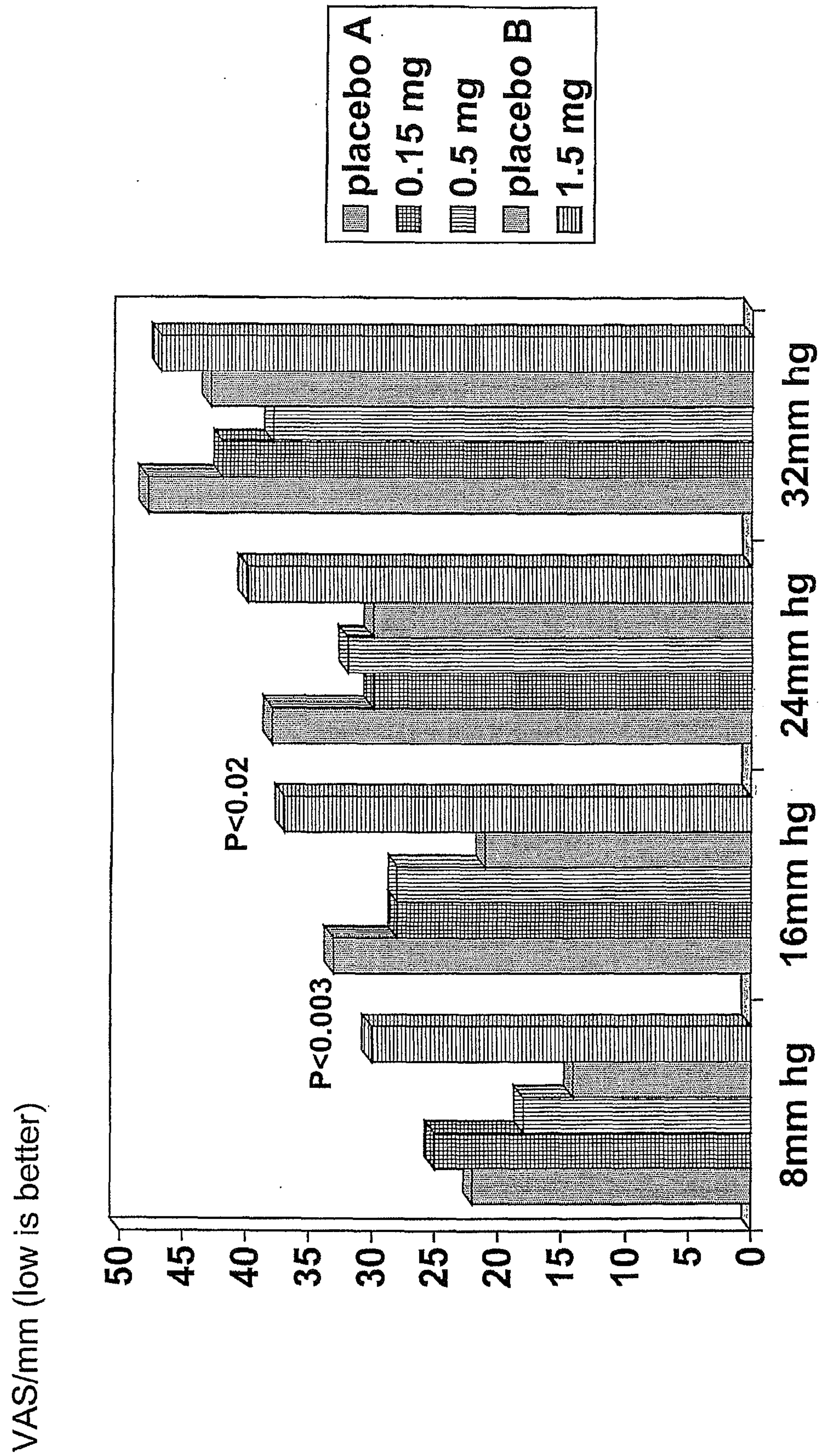
VAS/mm (low is better)
50
45
40
35
30
25
20
15
10
5
0
8mm hg
16mm hg
24mm hg
32mm hg
P<0.003
P<0.02
placebo A
0.15 mg
0.5 mg
placebo B
1.5 mg

Europäisches Patentamt
European Patent Office
Office européen des brevets

# PARTIAL EUROPEAN SEARCH REPORT

which under Rule 63 of the European Patent Convention shall be considered, for the purposes of subsequent proceedings, as the European search report

Application Number
EP 09 15 8047

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | WO 02/13801 A (CPD LLC)<br>21 February 2002 (2002-02-21)<br>* claims 1,5,15 *<br>----- | 1-3,5,18<br>1-7,18, 19 | INV.<br>A61K31/445<br>A61K31/40<br>A61K31/485<br>A61K31/135<br>A61P3/04 |
| X<br>Y | US 4 889 860 A (RZESZOTARSKI WACLAW J ET AL) 26 December 1989 (1989-12-26)<br>* claims 1,11,21 *<br>* column 1, line 14 - line 15 *<br>----- | 1-3,6,18<br>1-7,18, 19 | |
| X<br>Y | WO 01/98267 A (FENWICK ASHLEY EDWARD ;GETHIN DAVID MORRIS (GB); GIBSON STEPHEN PA) 27 December 2001 (2001-12-27)<br>* page 1, line 3 - line 8 *<br>----- | 1,2,6, 18-21<br>1-7,20, 21 | |
| E | WO 03/048113 A (SENANAYAKE CHRIS HUGH ;CURRIE MARK (US); GROVER PAUL T (US); FANG) 12 June 2003 (2003-06-12)<br>* page 2, paragraph [0006] *<br>* page 8, paragraph [0023] *<br>-----<br>-/-- | 1,2,6, 18-21 | |

TECHNICAL FIELDS SEARCHED (IPC)

A61K

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 5 May 2009 | Baurand, Petra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

3

EPO FORM 1503 03.82 (P04E07)

Europäisches Patentamt
European Patent Office
Office européen des brevets

# PARTIAL EUROPEAN SEARCH REPORT

Application Number
EP 09 15 8047

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| E | US 6 569 449 B1 (SWAAN PETER W ET AL) 27 May 2003 (2003-05-27) * claim 1 * * column 1, line 10 * ----- | 1-6, 18-21 | |
| X | MORLEY J E ET AL: "EFFECT OF BUTORPHANOL TARTRATE ON FOOD AND WATER CONSUMPTION IN HUMANS" AMERICAN JOURNAL OF CLINICAL NUTRITION, vol. 42, no. 6, 1985, pages 1175-1178, XP008022098 ISSN: 0002-9165 | 1,2,6,20 | |
| Y | * page 1177, left-hand column, last paragraph to right-hand column, 1st paragraph * ----- | 1-6,20, 21 | TECHNICAL FIELDS SEARCHED (IPC) |
| X | MENDELSON SCOTT D: "Treatment of anorexia nervosa with tramadol." AMERICAN JOURNAL OF PSYCHIATRY, vol. 158, no. 6, June 2001 (2001-06), pages 963-964, XP008022097 ISSN: 0002-953X * page 963, right-hand column, paragraph 1 * * page 964, left-hand column, paragraph 1 * ----- | 1-3,6,20 | |
| A | JOHNSON R D: "OPIOID INVOLVEMENT IN FEEDING BEHAVIOUR AND THE PATHOGENESIS OF CERTAIN EATING DISORDERS" MEDICAL HYPOTHESES, EDEN PRESS, PENRITH, US, vol. 45, no. 5, November 1995 (1995-11), pages 491-497, XP008016540 ISSN: 0306-9877 * entire document * ----- | | |

3

EPO FORM 1503 03.82 (P04C10)

Europäisches Patentamt
European Patent Office
Office européen des brevets

**INCOMPLETE SEARCH SHEET C**

Application Number
EP 09 15 8047

Reason for the limitation of the search:

Present claims 1 - 4, 6 - 9, 11, 12, 14 - 21 relate to a compound defined by reference to a desirable characteristic or property, namely "selective opiate receptor modulator", "receptor agonist", "appetite depressant" and "sympathomimeticum". The claims cover all compounds having this characteristic or property, whereas the application provides support within the meaning of Article 6 PCT and disclosure within the meaning of Article 5 PCT for only a very limited number of such compounds. In the present case, the claims so lack support, and the application so lacks disclosure, that a meaningful search over the whole of the claimed scope is impossible. Independent of the above reasoning, the claims also lack clarity (Article 6 PCT). An attempt is made to define the compound by reference to a result to be achieved. Again, this lack of clarity in the present case is such as to render a meaningful search over the whole of the claimed scope impossible. Consequently, the search has been carried out for those parts of the claims which appear to be clear, supported and disclosed, namely those parts relating to the the following compounds: Alvimopan, Loperamide, Asimadoline, Fedotozine, Pentazocine, U62066E, ICI204448, U-50488H, ADL 10-0101, ADL 10-0116, ADL 1-0398, Phenylpropanolamin, Cathin, Sibutramin, Amfepramon, Ephedrin and Norpseudoephedrin.

**ANNEX TO THE EUROPEAN SEARCH REPORT ON EUROPEAN PATENT APPLICATION NO.** EP 09 15 8047

This annex lists the patent family members relating to the patent documents cited in the above-mentioned European search report.
The members are as contained in the European Patent Office EDP file on
The European Patent Office is in no way liable for these particulars which are merely given for the purpose of information.

05-05-2009

| Patent document cited in search report | Publication date | Patent family member(s) | Publication date |
|---|---|---|---|
| WO 0213801 A | 21-02-2002 | AU 7695201 A | 25-02-2002 |
| | | EP 1357907 A2 | 05-11-2003 |
| | | US 6262062 B1 | 17-07-2001 |
| | | US 2002045636 A1 | 18-04-2002 |
| US 4889860 A | 26-12-1989 | NONE | |
| WO 0198267 A | 27-12-2001 | AR 028969 A1 | 28-05-2003 |
| | | AU 781837 B2 | 16-06-2005 |
| | | AU 6259101 A | 02-01-2002 |
| | | BG 107329 A | 31-07-2003 |
| | | BR 0111867 A | 01-07-2003 |
| | | CA 2412188 A1 | 27-12-2001 |
| | | CN 1639121 A | 13-07-2005 |
| | | CZ 20023967 A3 | 18-02-2004 |
| | | EA 5117 B1 | 28-10-2004 |
| | | EP 1292574 A1 | 19-03-2003 |
| | | HR 20020998 A2 | 29-02-2004 |
| | | HU 0301228 A2 | 28-08-2003 |
| | | IS 6637 A | 28-11-2002 |
| | | JP 2004512263 T | 22-04-2004 |
| | | MA 26915 A1 | 20-12-2004 |
| | | MX PA02012878 A | 14-05-2003 |
| | | NO 20026168 A | 18-02-2003 |
| | | NZ 523141 A | 24-06-2005 |
| | | OA 12293 A | 18-03-2004 |
| | | PA 8519401 A1 | 30-12-2002 |
| | | PL 365956 A1 | 24-01-2005 |
| | | SK 17172002 A3 | 08-09-2004 |
| | | UA 73176 C2 | 17-03-2003 |
| | | YU 91802 A | 25-05-2006 |
| | | ZA 200210278 A | 19-12-2003 |
| WO 03048113 A | 12-06-2003 | AU 2002364517 A1 | 17-06-2003 |
| | | US 2003171440 A1 | 11-09-2003 |
| US 6569449 B1 | 27-05-2003 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- DE 3935371 A1
- DE 4034785
- DE 4215231 A
- EP 0569802 A
- EP 0752246 A


### Non-patent literature cited in the description


- **J. N. Sengupta et al.** *Pain,* 1990, vol. 79, 175-185
- **Laurent Diop et al.** *European Journal of Pharmacology,* 1994, vol. 271, 65-71
- **Gottschlich et al.** *Chirality,* 1994, vol. 6, 685-689
- **Gottschlich et al.** *Drugs Exptl. Clin. Res.,* 1995, vol. XXI (5), 171-174
- **A. Barber et al.** *J. Pharmacol.,* 1994, vol. 113, 1317-1327
- **J. N. Junien ; P. Riviere.** *Aliment. Pharmacol. Ther,* 1995, vol. 9, 117-126
- **Krimmer, E. C. et al.** *Fed. Proc.,* vol. 41 (7), 2319-22
- **Spetea et al.** *Life Sciences,* 2001, vol. 69, 1775-1782
- **Lathi et al.** *European Journal Pharmacology,* 1985, vol. 109, 281-284
- *Am. J. Surg.,* November 2001, vol. 182 (5A), 27S-38S
- *J Pharmacol Exp Ther,* April 1999, vol. 289 (1), 494-502
- *Pol. J. Pharmacol.,* January 1994, vol. 46 (1-2), 37-41
- *Expert Opin Investig Drugs,* January 2001, vol. 10 (1), 97-110
- *Biol Pharm Bull.,* November 1997, vol. 20 (11), 1193-8
- *Br J Pharmacol.,* August 1992, vol. 106 (4), 783-9
- *Life Sci.,* 01 March 2002, vol. 70 (15), 1727-40
- *Pain,* 2002, vol. 96 (1-2), 13-22
- *Int. J. Pharm.,* 1995, vol. 115, 61-67